Europäisches Patentamt

European Patent Office

Office européen des brevets

(11) Veröffentlichungsnummer: **0 310 911**
**A2**

## (12) EUROPÄISCHE PATENTANMELDUNG

(21) Anmeldenummer: **88115851.3**

(22) Anmeldetag: **27.09.88**

(51) Int. Cl.4: **C12N 5/02**

(30) Priorität: **05.10.87 DE 3733636**

(43) Veröffentlichungstag der Anmeldung:
**12.04.89 Patentblatt 89/15**

(84) Benannte Vertragsstaaten:
**AT BE CH FR GB IT LI NL SE**

(71) Anmelder: **Schott Glaswerke**
**Hattenbergstrasse 10**
**D-6500 Mainz(DE)**

(84) **BE CH FR IT LI NL SE AT**

(71) Anmelder: **Carl-Zeiss-Stiftung trading as**
**SCHOTT GLASWERKE**
**Hattenbergstrasse 10**
**D-6500 Mainz 1(DE)**

(84) **GB**

(72) Erfinder: **Schnabel, Roland, Dr.**
**Breckenheimer Strasse 71**
**D-6238 Hofheim/Ts(DE)**
Erfinder: **Langer, Peter, Dr.**
**Junkerstrasse 43**
**D-6200 Wiesbaden-Nordenstadt(DE)**
Erfinder: **Lathan, Bernd, Dr.**
**Auf der Aspel 70**
**D-5000 Köln(DE)**

(74) Vertreter: **Schmitz, Waldemar, Dipl.-Phys.**
**Lessingstrasse 10**
**D-6200 Wiesbaden(DE)**

(54) Verfahren zum Vermehren von Zellen, insbesondere bei der Prüfung von Behandlungssubsstanzen auf Wirksamkeit.

(57) Verfahren zum Vermehren von Zellen, bei dem vereinzelte Zellen in eine poröse Glaskapillare eingebracht und darin immobilisiert werden und die Glaskapillare unter Vermehrungsbedingungen in einem Nährmedium gehalten wird, wobei die Dichte der Poren so groß ist wie ohne störende Einbuße an Festigkeit der Kapillare möglich, dadurch gekennzeichnet, daß für eine gegebene Sorte Zellen eine Kapillare verwendet wird, die einen zur Erzielung eines etwa maximalen Klonierungs-Wirkungsgrades gewählten Durchmesser hat.

EP 0 310 911 A2

# Verfahren zum Vermehren von Zellen, insbesondere bei der Prüfung von Behandlungssubstanzen auf Wirksamkeit

Die Erfindung betrifft ein Verfahren zum Vermehren von Zellen, insbesondere bei der Prüfung von Behandlungssubstanzen auf Wirksamkeit durch Beobachtung der Hemmung der Vermehrung durch die Behandlungssubstanz, bei welchem Verfahren eine Anzahl von vereinzelten Zellen in eine poröse Glaskapillare eingebracht und in der Glaskapillare immobilisiert wird und die Glaskapillare unter Vermehrungsbedingungen in einem Nährmedium gehalten wird, wobei die Dichte der Poren der Glaskapillare so groß ist wie ohne störende Einbuße an Festigkeit der Kapillaren möglich, wobei ferner vorzugsweise zur Immobilisierung eine mit einer Immobilisierungssubstanz beschichtete Kapillare verwendet wird und vorzugsweise die Anzahl der eingebrachten Zellen in Abhängigkeit von dem Volumen der Kapillare zur Erzielung eines etwa maximalen Klonierungs-Wirkungsgrades gewählt wird.

Die kontrollierte Haltung, Vermehrung und Beeinflussung von tierischen oder planzlichen Zellkulturen einschließlich Gewebe- und/oder Organkulturen ist die Voraussetzung für deren industriellen oder medizinischen Einsatz. Insbesondere für Zwecke der Diagnostik oder bei der Prüfung von Behandlungsmitteln auf Wirksamkeit ist eine rasche Vermehrung erwünscht, um möglichst schnell verwendbare Ergebnisse zu erhalten. Aus diesen und anderen Gründen besteht ein wesentliches Ziel der Zellkultivierung in der Aufrechterhaltung und vorzugsweise Beschleunigung der Proliferation.

Für die Kultivierung und Vermehrung von Zellen ist eine Anzahl von unterschiedlichen Techniken bekannt.

Für Zwecke der Diagnostik und der Prüfung der Wirkung von Behandlungsmitteln ist es vorteilhaft, wenn die eingebrachten Zellen immobilisiert werden und dadurch vereinzelt bleiben. Zu diesem Zweck werden in der Regel die Zellen an Oberflächen immobilisiert, und um die Anzahl der einbringbaren Zellen zu erhöhen, sind folgerichtig Zellkultur-Behälter entwickelt worden, bei denen durch Einbauten, zum Beispiel Rohrschlangen, die Oberfläche im Verhältnis zum Volumen vergrößert ist. Stattdessen kann man auch die sogenannte Mikroscannertechnik anwenden (EP 58689), bei der Zellen in einer Suspensionskultur vereinzelt gehalten werden.

Bei allen Verfahren, bei denen Zellen in verhältnismäßig hoher Dichte eingebracht werden, kann die Versorgung mit Nährstoffen und die Entfernung von Metaboliten Schwierigkeiten bereiten, ferner kann dabei die weitere Schwierigkeit auftreten, daß die Metaboliten wachstumshemmende und/oder wachstumsfördernde Komponenten enthalten können.

Bei körperlicher Trennung von Nährmedium und Zellkultur durch eine semipermeable Membran (P.M. Guilino, R.A. Knazek: Tissue culture on artificial capillaries; Methods in enzymology 58, Cell Culture, Academic Press, Seiten 178-184 (1979)) sind die austauschbaren Stoffströme verhältnismäßig klein. Im Gegensatz dazu ist bei dem eingangs beschriebenen Verfahren (EP 183 207) über die Kapillaren ein direkter, nicht von einer semipermeablen Membran behinderter Stoffaustausch möglich. Dennoch sind die erzielbaren Vermehrungsraten oder Klonierungs-Wirkungsgrade unbefriedigend, so beträgt zum Beispiel der Klonierungs-Wirkungsgrad, d.h. das Verhältnis der Anzahl der gebildeten Zellkulturen (je mehr als 50 Zellen) zu der Anzahl der eingebrachten Zellen nach 14 Tagen Inkubationszeit typischerweise nur Bruchteile eines Prozents, auch wenn in Anwendung der Erkenntnis, daß der Klonierungs-Wirkungsgrad von der Konzentration der eingebrachten Zellen abhängt, die Anzahl der eingebrachten Zellen so gewählt wird, daß sich bei den gegebenen Abmessungen der Kapillare ein etwa maximaler Klonierungs-Wirkungsgrad ergibt (D.D. von Hoff und Mai Hoang, AACR Abstracts 1983, 310, Nr. 1225).

Die vorliegende Erfindung geht von der Aufgabe aus, ein einfaches Verfahren zum Vermehren von Zellen zu schaffen, mit dem hohe Poliferationsraten erzielbar sind.

Nach der Erfindung wird diese Aufgabe gelöst mit einem Verfahren der eingangs angegeben Art, das dadurch gekennzeichnet ist, daß für eine gegebene Sorte Zellen eine Kapillare verwendet wird, die einen zur Erzielung eines etwa maximalen Klonierungs-Wirkungsgrades gewählten Porendurchmesser hat.

Erfindungsgemäß ist gefunden worden, daß der Klonierungs-Wirkungsgrad (oder allgemein die Vermehrungsrate) in einem verhältnismäßig engen Bereich von Porengrößen ein Maximum hat. Verwendet man eine Kapillare mit Poren, deren Größe in diesem Bereich liegt, so ergeben sich Klonierungs-Wirkungsgrade, die mehr als eine Größenordnung größer sind als bei bekannten Verfahren. Dadurch kann es in verhältnismäßig kurzen Inkubationszeiten, jedenfalls aber in üblichen Inkubationszeiten von z.B. 14 Tagen, zur Bildung so großer Zellkolonien kommen, daß die Zellkolonien direkt mikroskopisch beobachtbar und auswertbar sind, wobei auch eine maschinelle und/oder automatische Beobachtung und Auswertung stark erleichtert ist.

Das erfindungsgemäße Verfahren bietet besonders große Vorteile bei der Prüfung von Behandlungssubstanzen bezüglich ihrer Wirkung auf Zellen, z.B. Karzinomzellen, die beispielsweise von einem Biopsie- oder Operationspräparat stammen. Die hohe Vermehrungsrate führt zu entsprechend deutlichen und leicht unterscheidbaren und auswertbaren Prüfergebnissen.

Überraschenderweise hat sich gezeigt, daß für die meisten Zellarten eine Kapillare mit einer Wanddicke zwischen etwa 20 und 200 µm und einer Porengröße zwischen etwa 6 und 50 nm, vorzugsweise 8 und 30 nm, die beschriebenen günstigen Ergebnisse erbringt.

Es hat sich ferner gezeigt, daß in einem bestimmten Bereich des Verhältnisses von Oberfläche zu Volumen der Kapillare ein besonders hoher Maximalwert des Klonierungs-Wirkungsgrades enthalten wird. Dieses Verhältnis ergibt sich, wenn man eine Kapillare mit der angegebenen Wanddicke und einem Außendurchmesser von 0,2 bis 4 mm verwendet.

Die Erfindung wird im folgenden anhand von Beispielen weiter erläutert.

Beispiel 1

Von der menschlichen Mammakarzinom-Zellinie MDA-231 wurde enzymatisch eine Einzelzellsuspension erstellt. Die Zellen (in der Regel 2000/ml) wurden dann in MEM-Medium mit 10 % FCS + 25 mMol HEPES und 0,3 % Agar suspendiert.

Mehrere poröse Kapillaren unterschiedlicher Porengröße wurden steril mit je 50 bis 75 µl Zellsuspension und Agar gefüllt und anschließend in Perfusionsgefäße (Röhrchen) mit 10 ml Medium (s. oben), aber ohne Agar, eingelegt. Nach 14 Tagen Inkubationszeit Auswertung unter einem Invertmikroskop, wobei die Kapillaren in 0,9 % NaCl-Lösung oder Medium gelegt werden, so daß optische Störungen durch Brechungsindex-Unterschiede entsprechend gering sind.

Die Tabelle 1 gibt die Klonierungs-Wirkungsgrade von MDA-231-Zellen in perfundierten porösen Kapillaren unterschiedlicher Prengröße an. Der Klonierungs-Wirkungsgrad (cloning efficiency) ist das Verhältnis der nach einer gegebenen Inkubationszeit, meist 14 Tagen, vorliegenden Anzahl von Zellkolonien (je mehr als 50 Zellen) zu der Anzahl der anfänglich eingebrachten vereinzelten Zellen. Man erkennt, daß bei Porengrößen im Bereich um 13 nm ein ausgeprägtes Maximum des Klonierungs-Wirkungsgrades vorliegt.

Beispiel 2

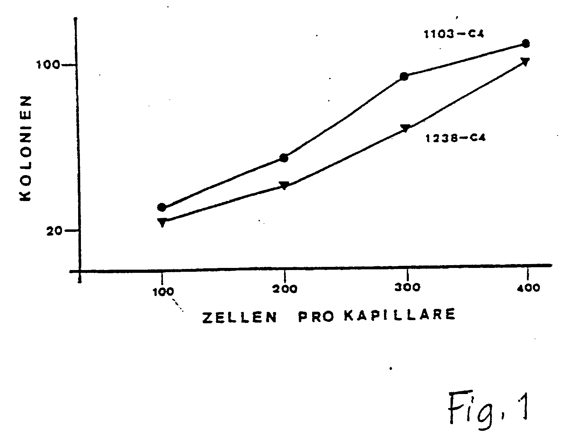

Bei dem erfindungsgemäßen Verfahren kommt es auf die Konzentration an Zellen in der Kapillare weniger an. Um dies nachzuweisen, wurden in zwei verschiedenen Kapillaren verschiedene Anzahlen von Zellen des genannten Typs MDA-231 eingebracht. Figur 1 zeigt, daß nach 14-tägiger Inkubationszeit die Anzahl der gebildeten Kolonien nach einer linearen Funktion von der Anzahl der eingebrachten Zellen abhängt. Dies ist von großer Bedeutung für Prüfungen von Behandlungsmitteln, z.B. Zytostatika.

Die Beschichtung der Kapillaren mit einer geeigneten Immobilisierungssubstanz kann bedeutungsvoll sein; so kann es vorkommen, daß bei bestimmten Zellsorten ohne eine solche immobilisierende Beschichtung überhaupt keine Vermehrung eintritt.

Besonders gut geeignet ist das aus der DE-OS 24 62 567 bekannte Silanisierungsverfahren, wobei entweder in der Primärreaktion direkt eine funktionelle Gruppe wie $NH_2OH$, $SO_3H$, $COOH$, $NO_2$, $CH_3$, $CH_2Cl$, $N^+$ als neue Oberflächengruppe eingebracht werden kann oder aber auch in einer Folgereaktion die Oberfläche zu einer bioaktiven Oberfläche geändert werden kann. Derartige Reaktionen können z.B. zu einer Polyethylenimin-Oberfläche, zu einer DEAE-Oberfläche oder auch PEG-Oberfläche führen. Die Sekundärreaktion kann andererseits auch mit oder ohne Spacer zu einer enzymatisch aktiven Oberfläche oder durch Verwendung spezifischer Liganden wie z.B. Protein A oder monoklonale Antikörper zu einer substanzspezifischen Oberfläche führen.

Die Länge der Kapillare ist nicht von ausschlaggebender Bedeutung. Zweckmäßig ist eine Kapillare mit einer Länge zwischen 2 und 100 cm.

In Bezug auf die Konzentration der Zellen ist es vorteilhaft, wenn die Zellen mit einer Konzentration von etwa $2000/cm^3$ eingebracht werden. Dabei liegt noch eine ausreichende Vereinzelung vor. Vorzugsweise werden etwa 100 bis 500 Zellen in die Kapillare eingebracht.

Für die Durchführung des erfindungsgemäßen Verfahrens sind insbesondere poröse Kapillaren geeignet, die nach den Verfahren gemäß den deutschen Offenlegungsschriften 24 54 111 und 24 62 567 hergestellt worden sind. Es können aber auch auf andere Weise hergestellte poröse Glaskapillaren verwendet werden. Das genannte Verfahren zeigt allerdings deutliche Vorteile in Bezug auf Homogenität der Zusammensetzung und der kontrollierbaren Porenverteilung. Wesentlich ist, daß die Abmessungen der Kapillaren und die Porengrößen in den oben angegebenen Grenzen liegen. Da die Oberfläche von porösen Gläsern äußerst reaktiv ist, ist es zweckmäßig, zur Erzielung eines kontrollierbaren Zellwachstums bzw. einer kontrollierbaren Kolonienbildung die Oberflächenaktivität der Polarität der Zellen anzupassen. Hierfür wird vorzugsweise das aus der DE-OS 24 62 567 bekannte Silanisierungsverfahren eingesetzt, das weiter vorn bereits besprochen wurde.

Tabelle 1

Klonierungs-Wirkungsgrade (Cloning efficiencies) von menschlichen Mamma-Carcinom-Zellen MDA-231 in perfundierten porösen Kapillaren unterschiedlicher Porengröße

| Kapillare | Porengröße $\tilde{x}$) $r_{90}$ (nm) | Cloning* Efficiency (%) | Mechan. xx) Stabilität |
|---|---|---|---|
| 1103-A4 | 5,3 | $\emptyset$ | + |
| 1103-B4 | 5,9 | $\emptyset$ | + |
| 1238-B4 | 6,5 | 23,3 | - |
| 1549-210 | 6,9 | 26,1 | + |
| 1550-D4 | 7,4 | 27,4 | + |
| 1103-C4 | 8,8 | 27,2 | + |
| 1238-C4 | 10,6 | 39,6 | + |
| 1550-110 | 13,5 | 49,1 | + |
| 1550-310 | 15,4 | 31,6 | + |
| 1553-220 | 15,5 | 24,9 | (+) |
| 1235-B4 | 15,7 | 18,9 | + |
| 1235-A4 | 15,8 | 29,0 | - |
| 1236-A4 | 17,3 | 9,1 | (+) |
| 1237-A4 | 27,9 | 12,3 | (+) |

*) Klonierungs-Wirkungsgrad:

$$\frac{\text{Anzahl Kolonien} (> 50 \text{ Zellen}) \text{ nach 14 Tagen}}{\text{Anzahl eingebrachter Zellen}} \times 100$$

xx) Es bedeuten :

+ zufriedenstellend

(+) weniger gut

- sehr zerbrechlich

x) Porenradius, gemessen durch Quecksilber-Porosimetrie

**Ansprüche**

1. Verfahren zum Vermehren von Zellen, insbesondere bei der Prüfung von Behandlungssubstanzen auf Wirksamkeit durch Beobachtung der Hemmung der Vermehrung durch die Behandlungssubstanz, bei welchem Verfahren eine Anzahl von vereinzelten Zellen in eine poröse Glaskapillare eingebracht und in der Glaskapillare immobilisiert wird, und die Glaskapillare unter Vermehrungsbedingungen in einem Nährmedium gehalten wird, wobei die Dichte der Poren der Kapillare so groß ist wie ohne störenden Einfluß an Festigkeit der Kapillare möglich, wobei ferner vorzugsweise zur Immobilisierung eine mit einer Immobilisierungssubstanz beschichtete Kapillare verwendet wird, und vorzugsweise die Anzahl der eingebrachten Zellen in Abhängigkeit von dem Volumen der Kapillare zur Erzielung eines etwa maximalen Klonierungs-Wirkungsgrades gewählt wird, dadurch gekennzeichnet, daß eine Kapillare mit einer Wanddicke zwischen 20 und 200 μm, einer Porengröße zwischen 6 und 50 nm und einem Durchmesser von 0,2 bis 4 mm verwendet wird.

2. Verfahren nach Anspruch 1, dadurch gekennzeichnet, daß eine Kapillare mit einer Porengröße zwischen 8 und 30 nm verwendet wird.

3. Verfahren nach Anspruch 1 oder 2, dadurch gekennzeichnet, daß eine Kapillare mit einer Länge zwischen 2 und 100 cm verwendet wird.

4. Verfahren nach einem der Ansprüche 1 bis 3, dadurch gekennzeichnet, daß die Zellen mit einer Konzentration von etwa 2000/cm$^3$ eingebracht werden.

5. Verfahren nach einem der Ansprüche 1 bis 4, dadurch gekennzeichnet, daß etwa 100 bis 500 Zellen in die Kapillare eingebracht werden.

6. Verwendung einer porösen Glaskapillare mit einem äußeren Durchmesser von 0,2 bis 4 mm, einer Wanddicke von 20 bis 200 μm, einer Länge von 2 bis 100 cm und Porendurchmessern von 6 bis 50 nm bei einem Verfahren nach einem der vorhergehenden Ansprüche.

7. Verwendung nach Anspruch 6, dadurch gekennzeichnet, daß die Glaskapillare einen Porendurchmesser von 8 bis 30 nm aufweist.

Fig. 1